# EUROPEAN PATENT SPECIFICATION

(11) **EP 1 286 672 B1**
(45) Date of publication and mention of the grant of the patent: **25.10.2006**
(21) Application number: 01932011.8
(22) Date of filing: 28.05.2001
(51) Int. Cl.: A61K 31/496, A61P 27/02, A61P 27/06

(54) **S-methyl-dihydro-ziprasidone for the treatment of ocular disorders.**
S-methyl-dihydro-ziprasidon zur Behandlung von Augenerkrankungen.
S-methyl-dihydro-ziprasidone pour traiter des troubles oculaires.

(30) Priority: 02.06.2000 US 209136 P; 16.06.2000 US 212172 P
(43) Date of publication of application: 05.03.2003
(60) Divisional application: 04013380.3
(73) Proprietor: Pfizer Products Inc., Groton, Connecticut 06340 (US)
(72) Inventor: PRAKASH, Chandra A., Pfizer Global Research & Dev., Groton, CT 06340 (US); SMOLAREK, Teresa A., Pfizer Global Research & Dev., Groton, CT 06340 (US)
(74) Representative: Hayles, James Richard
(86) International application number: PCT/IB2001/000933
(87) International publication number: WO 2001/091756

(56) References cited:
- EP-A- 0 985 414
- WO-A-00/59489
- PRAKASH C ET AL: "METABOLISM AND EXCRETION OF A NEW ANTIPSYCHOTIC DRUG, ZIPRASIDONE, IN HUMANS" DRUG METABOLISM AND DISPOSITION, WILLIAMS AND WILKINS., BALTIMORE, MD, US, vol. 25, no. 7, July 1997 (1997-07), pages 863-872, XP000987000 ISSN: 0090-9556
- MANO, T., ET. AL.: "The effect of Anplag (sarpogrelate HCl), novel selective 5-HT2 antagonist on intraocular pressure in rabbits." INVESTIGATIVE OPHTHALMOLOGY AND VISUAL SCIENCE, vol. 36, 1995, page 719 XP008024786
- TAKANEKA, H., ET.AL.: "The effect of Anplag (sarpogrelate HCl), novel selective 5-HT2 antagonist on intraocular pressure in glaucoma patients." INVESTIGATIVE OPHTHALMOLOGY AND VISUAL SCIENCE, vol. 36, 1995, page 734 XP008024785
- SEEGER T F ET AL: "Ziprasidone (CP-88,059): a new antipsychotic with combined dopamine and serotonin receptor antagonist activity." THE JOURNAL OF PHARMACOLOGY AND EXPERIMENTAL THERAPEUTICS. UNITED STATES OCT 1995, vol. 275, no. 1, October 1995 (1995-10), pages 101-113, XP002051730 ISSN: 0022-3565

## Description

### Background of the Invention

This invention relates to the use of S-methyl-dihydroziprasidone and its pharmaceutically acceptable salts for the treatment of ocular disorders. More specifically, it relates to the use of such compound and its pharmaceutically acceptable salts for the treatment of a disorder or condition selected from: glaucoma; and ischemic retinopathy.

S-methyl-dihydro-ziprasidone, which has the following structure, 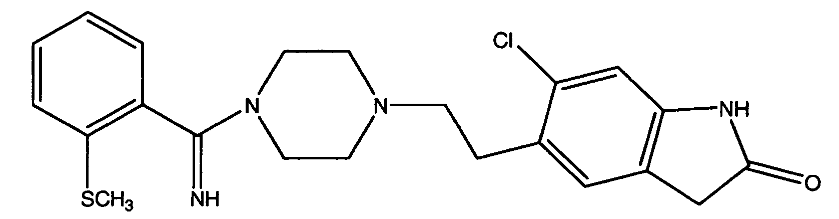 and the chemical name 6-chloro-5-(2-{4-[imino-(2-methylsulfanylphenyl)-methyl]-piperazin-1-yl}-ethyl)-1,3-dihydro-indol-2-one, is an active metabolite of the antipsychotic drug ziprasidone, which has the following structure

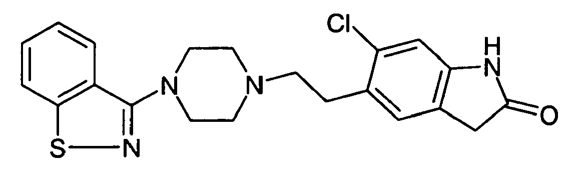

Ziprasidone and related arylpiperazinyl-(C₂-C₄)alkylene-heterocyclic compounds, methods for their synthesis and their use in the treatment of psychotic disorders of the schizophrenic types and for removing or ameliorating such symptoms as anxiety, agitation, excessive aggression, tension and social or emotional withdrawal in psychotic patients are referred to in United States Patent 4,831,031, which issued on May 16, 1989, United States Patent 5,206,366, which issued on April 27, 1993, United States Patent 4,833,795, which issued on November 28, 1989, United States Patent 5,312,925, which issued on May 17, 1994 and United States Patent 5,338,846, which issued on August 16, 1994. The use of ziprasidone and such related compounds for the treatment of obsessive-compulsive disorder and Tourette's syndrome is referred to in United States Patent Application 09/146,289, which was filed on September 3, 1998. The use of ziprasidone and such related compounds for the treatment of behavioral symptoms associated with psychosis is referred to in United States Patent Application 09/216,344, which was filed on December 8, 1998. The use of ziprasidone and such related compounds for the treatment of glaucoma and ischemic retinopathy is referred to in United States Patent Application 09/314,792, which was filed on May 18, 1999.

### Summary of the Invention

This invention relates to use of 6-chloro-5-(2-{4-[imino-(2-methylsulfanylphenyl)-methyl]-piperazin-1-yl}-ethyl)-1,3-dihydro-indol-2-one, or a pharmaceutically acceptable salt thereof, for the manufacture of a medicament for the treatment of a disorder or condition selected from: glaucoma; and ischemic retinopathy. This use is hereinafter also referred to as "the inventive use".

The term "treating", as used herein, refers to reversing, alleviating, inhibiting the progress of, or preventing the disorder or condition to which such term applies, or one or more symptoms of such disorders or condition. The term "treatment", as used herein, refers to the act of treating, as "treating" is defined immediately above.

The present invention also relates to the above inventive use, wherein a radiolabelled form of S-methyl-dihydro-ziprasidone is used instead of S-methyl-dihydro-ziprasidone. Preferred radiolabelled compounds of S-methyl-dihydro-ziprasidone are those wherein the radiolabels are selected from as ³H, ¹¹C, ²H, ¹³C, ¹⁴C, ¹⁸F, ¹²³I and ¹²⁵I. Such radiolabelled compounds are useful as research and diagnostic tools in metabolism pharmacokinetics studies and in binding assays in both animals and man.

Examples of pharmaceutically acceptable acid addition salts of the compounds of formula I are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Another more specific embodiment of this invention relates to the above inventive use wherein the disorder or condition being treated is glaucoma.

Another more specific embodiment of this invention relates to the above inventive use wherein the disorder or condition being treated is ischemic retinopathy.

### Detailed Description of the Invention

S-methyl-dihydro-ziprasidone can be prepared by reacting dihydro-ziprasidone with a methylating agent (*e.g.*, iodomethane or diazomethane), preferably with iodomethane. This reaction is preferably carried out under a nitrogen atmosphere. It is typically carried out in a lower alkanol solvent such as methanol, ethanol or propanol, preferably methanol, at a temperature from about 0°C to about the reflux temperature of the solvent, preferably at about room temperature, in the presence of a strong base such as potassium hydroxide, sodium hydroxide, potassium or sodium t-butoxide, or potassium or sodium carbonate.

Dihydro-ziprasidone can be prepared as described in United States Patent 5,935,960, which issued on August 10, 1999.

The pharmaceutically acceptable acid addition salts of S-methyl-dihydro-ziprasidone are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are the acetic, hydrochloric, hydrobromic, hydroiodic, nitric, sulfonic, sulfuric, isonicotinic, lactic, salicylic, citric, tartaric, pantothenic, bitartaric, ascorbic, succinic, maleic, fumaric, glucaronic, saccharate, formate, benzoate, glutamate, methanesulfonic, ethanesulfonic, benzenesulfonic, p-toluenesulfonic, and gluconic acids.

S-methyl-dihydro-ziprasidone and its pharmaceutically acceptable salts (referred to collectively, hereinafter, as "the active compounds of this invention"), can be administered to a human subject either alone, or, preferably, in combination with pharmaceutically acceptable carriers or diluents, in a pharmaceutical practice. Such compounds can be administered orally or parenterally. Parenteral administration includes especially intravenous and intramuscular administration. Additionally, in a pharmaceutical composition comprising an active compound of this invention, the weight ratio of active ingredient to carrier will normally be in the range from 1:6 to 2:1, and preferably 1:4 to 1:1. However, in any given case, the ratio chosen will depend on such factors as the solubility of the active component, the dosage contemplated and the precise route of administration.

The active compounds of this invention can be administered to mammals via either the oral, parenteral (such as subcutaneous, intravenous, intramuscular, intrasternal and infusion techniques), rectal, intranasal or topical routes. In general, these compounds are most desirably administered in doses ranging from 0.5 to 500 mg per day, in single or divided doses (i.e., from 1 to 4 doses per day), although variations will necessarily occur depending upon the species, weight and condition of the subject being treated and the particular route of administration chosen. However, a dosage level that is in the range of 10 mg to 80 mg per kg of body weight per day is most desirably employed. Nevertheless, variations may occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such higher dose levels are first divided into several small doses for administration throughout the day.

The active compounds of this invention can be administered alone or in combination with pharmaceutically acceptable carriers or diluents by any of the routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. Moreover, oral pharmaceutical compositions can be suitably sweetened and/or flavored. In general, the therapeutically-effective compounds of this invention are present in such dosage forms at concentration levels ranging from 5.0% to 70% by weight.

For oral use in treating the various disorders and conditions referred to above, the can be administered, for example, in the form of tablets or capsules, or as an aqueous solution or suspension. Tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

For intramuscular, parenteral and intravenous use, sterile solutions of the active ingredient can be prepared, and the pH of the solutions should be suitably adjusted and buffered. For intravenous use, the total concentration of solutes should be controlled to render the preparation isotonic.

Additionally, it is also possible to administer the compounds of the present invention topically when treating ocular disorders such as glaucoma and ischemic retinopathy and this may be done by way of creams, jellies, gels, pastes, patches, ointments and the like, in accordance with standard pharmaceutical practice.

The following example illustrates the synthesis of S-methyl-dihydro-ziprasidone.

### EXAMPLE 1

### 6-CHLORO-5-(2-{4-[IMINO-(2-METHYLSULFANYLPHENYL)-METHYL]-PIPERAZIN-1-YL}-ETHYL)-1,3-DIHYDRO-INDOL-2-ONE

Potassium hydroxide (1.82 grams) was added to methanol (1300 ml) under a nitrogen atmosphere and stirred for 15 minutes at 21°C. Dihydro-ziprasidone (13.0 grams, 31.3 mmol) was added and the mixture was stirred at 21°C until a solution was formed. Iodomethane (2.34 ml, 37.7 mmol, 1.2 equivalents) was added and the solution was allowed to stir overnight at 21°C. The progress of the reaction was followed using thin layer chromatography (silica gel, eluting with 4:1 methylene chloride:isopropanol, visualizing with UV lamp at 254 nm). The reaction mixture was vacuum concentrated and 2.0 liters of methylene chloride and 500 ml of water were added to the remaining solid. The hazy mixture was allowed to stir for 15 minutes, after which the water layer (pH=9.8) was discarded. The methylene chloride layer, which was light pink, was dried using magnesium sulfate and Darco G60 was added to decolorize the solid. The mixture was allowed to stir for another 15 minutes at 21 °C and filtered over Celite. The Celite filter pad was washed with 50 ml methylene chloride and the combined filtrates were vacuum concentrated to a solid (13.05 grams) that was a lighter pink. Further purification using flash chromatography with silica gel and a methylene chloride eluent yielded 7.38 grams of the title compound. M.P. 103 - 106°C (gassing). Mass spectrum (m/e, % intensity): 429 (100, M+1), 236,194. ¹³C NMR (CDCl3) 177.70, 166.83, 142.36, 136.94, 136.17, 133.10, 131.30, 129.51, 126.86, 125.27, 124.46, 111.06, 58.84, 53.11, 36.09, 30.95 and 15.87.

## Claims

1. Use of 6-chloro-5-(2-{4-[imino-(2-methylsulfanylphenyl)-methyl]-piperazin-1-yl}-ethyl)-1,3-dihydro-indol-2-one, or a pharmaceutically acceptable salt thereof, for the manufacture of a medicament for the treatment of a disorder or condition selected from: glaucoma; and ischemic retinopathy.

2. Use according to Claim 1, wherein the disorder or condition being treated is glaucoma.

3. Use according to Claim 1, wherein the disorder or condition being treated is ischemic retinopathy.

## Patentansprüche

1. Verwendung von 6-Chlor-5-(2-{4-[imino-(2-methylsulfanylphenyl)-methyl]-piperazin-1-yl}-ethyl)-1,3-dihydroindol-2-on oder eines pharmazeutisch akzeptablen Salzes desselben zur Herstellung eines Medikaments zur Behandlung einer Störung oder eines Zustands, der aus grünem Star und ischämischer Retinopathie ausgewählt ist.

2. Verwendung nach Anspruch 1, wobei die zu behandelnde Störung oder der zu behandelnde Zustand grüner Star ist.

3. Verwendung nach Anspruch 1, wobei die zu behandelnde Störung oder der zu behandelnde Zustand ischämische Retinopathie ist.

## Revendications

1. Utilisation de 6-chloro-5-(2-{4-[imino-(2-méthylsulfanylphényl)-méthyl]-pipérazine-1-yl}-éthyl)-1,3-dihydroindole-2-one ou d'un de ses sels pharmaceutiquement acceptables, pour la production d'un médicament destiné au traitement d'un trouble ou d'une affection choisi entre : le glaucome et la rétinopathie ischémique.

2. Utilisation suivant la revendication 1, dans laquelle le trouble ou l'affection qui est traité est le glaucome.

3. Utilisation suivant la revendication 1, dans laquelle le trouble ou l'affection qui est traité est la rétinopathie ischémique.
